Europäisches Patentamt

European Patent Office

Office européen des brevets

(19)

(11) Numéro de publication : **0 277 894 B1**

## (12) FASCICULE DE BREVET EUROPEEN

(45) Date de publication du fascicule du brevet :
**29.01.92 Bulletin 92/05**

(51) Int. Cl.$^5$ : **C07C 45/71,** C07C 45/64,
C07C 47/575

(21) Numéro de dépôt : **88420024.7**

(22) Date de dépôt : **27.01.88**

(54) **Procédé de préparation de trialkoxybenzaldéhyde.**

(30) Priorité : **28.01.87 FR 8701174**

(43) Date de publication de la demande :
**10.08.88 Bulletin 88/32**

(45) Mention de la délivrance du brevet :
**29.01.92 Bulletin 92/05**

(84) Etats contractants désignés :
**AT BE CH DE ES FR GB GR IT LI LU NL SE**

(56) Documents cités :
**GB-A- 1 338 890
J.A.C.S., vol. 74, 5 septembre 1952, pages
4262-4263; I.A. PEARL et al.: "Reactions of
vanillin and its derived compounds. XVII. A
synthesis of syringaldehyde from vanillin"**

(56) Documents cités :
**CHEMICAL ABSTRACTS, vol. 102, no. 17, avril
1985, page 571, résumé no. 148883k, Columbus, Ohio, US; & PL-A-126 462 (AKADEMIA
EKONOMICZNA WROCLAW) 15-09-1984
CHEMICAL ABSTRACTS, vol. 96, 1982, page
670, résumé no. 103857w, Columbus, Ohio, US;
& JP-A-81 150 034 (UBE INDUSTRIES LTD)
20-11-1981
SYNTHESIS, 1983, page 308; D.V. RAO et al.:
"An efficient synthesis of 3,4,5-trimethoxy-
benzaldehyde from vanillin"**

(73) Titulaire : **RHONE-POULENC CHIMIE
25, quai Paul Doumer
F-92408 Courbevoie Cédex (FR)**

(72) Inventeur : **Crochemore, Michel
3, rue des Lilas Domaine de Gilbertain
F-69630 Chaponost (FR)**

(74) Mandataire : **Dutruc-Rosset, Marie-Claude et
al
RHONE-POULENC CHIMIE Direction des
Brevets 25, Quai Paul Doumer
F-92408 Courbevoie Cédex (FR)**

## Description

La présente invention concerne un procédé amélioré de préparation de trialkoxy-3,4,5 benzaldéhyde et plus particulièrement de triméthoxy-3,4,5 benzaldéhyde à partir de bromo-5 hydroxy-4 méthoxy-3 benzaldéhyde.

L'état de la technique divulgue plusieurs techniques d'accès au triméthoxy-3,4,5 benzaldéhyde.

Il est connu selon Durvasula V. RAO et al [SYNTHESIS p.308 (1983)] de préparer le triméthoxy-3,4,5 benzaldéhyde selon un procédé qui consiste :

– à faire réagir la bromo-5 vanilline avec le méthylate de sodium, en présence d'un catalyseur au cuivre et dans un mélange de solvants ($CH_3OH$/DMF),

– à séparer l'hydroxy-4 diméthoxy-3,4 benzaldéhyde ainsi obtenu,

– puis à conduire une réaction d'éthérification à l'aide de sulfate de diméthyle.

Une autre voie plus courante d'obtention du triméthoxy-3,4,5 benzaldéhyde à partir de bromo-5 hydroxy-4 méthoxy-3 benzaldéhyde (ou bromo-5 vanilline) consiste à hydrolyser l'atome de brome à l'aide d'une solution aqueuse de soude en présence de cuivre.

Après isolement du dihydroxy-4,5 méthoxy-3 benzaldéhyde (ou hydroxy-5 vanilline) obtenu, celui-ci est méthylé à l'aide de sulfate de diméthyle ou de chlorure de méthyle.

Ainsi la demande de brevet français No. 2 177 693 décrit la méthylation de l'hydroxy-5 vanilline par du sulfate de diméthyle en présence d'un carbonate de métal alcalin.

L'hydroxy-5 vanilline est elle-même obtenue par hydrolyse de la bromo-5 vanilline, puis est extraite notamment par le toluène pendant 47 heures, recristallisée, lavée et séchée.

La phase d'isolement de l'hydroxy-5 vanilline est donc longue et coûteuse. En outre le catalyseur ne peut pas être récupéré et recyclé.

Il a maintenant été trouvé que l'on pouvait, avec un bon rendement, opérer l'hydrolyse de la bromo-5 vanilline et l'éthérification de l'hydroxy-5 vanilline ainsi obtenue successivement sans séparation intermédiaire de l'hydroxy-5 vanilline.

Un autre avantage du procédé de l'invention consiste en ce que le cuivre servant de catalyseur dans l'hydrolyse de la bromo-5 vanilline peut être récupéré et recyclé dans une opération ultérieure.

Plus précisément l'invention est un procédé de préparation de dialkoxy-4,5 méthoxy-3 benzaldéhyde à partir de bromo-5 hydroxy-4 méthoxy-3 benzaldéhyde, caractérisé en ce que l'on effectue successivement et sans séparation des composés intermédiaires :

– l'hydrolyse du bromo-5 hydroxy-4 méthoxy-3 benzaldéhyde en dihydroxy-4,5 méthoxy-3 benzaldéhyde, par un hydroxyde de métal alcalin, dans l'eau et en présence d'un catalyseur au cuivre ;

– l'éthérification du dihydroxy-4,5 méthoxy-3 benzaldéhyde obtenu précédemment à l'aide d'un halogénure d'alkyle inférieur, en milieu aqueux et en maintenant le pH entre 6 et 12, éventuellement en présence d'un catalyseur.

Lorsque l'on opère à un pH inférieur à 6, on observe une importante hydrolyse de l'halogénure d'alkyle et une diminution quantitative de l'éthérification ainsi qu'un ralentissement de cette réaction.

Tous les hydroxydes de métaux alcalins peuvent être utilisés dans la première réaction du procédé. Cependant pour des questions de prix la soude est préférée.

Habituellement on met en oeuvre au moins une mole d'hydroxyde de métal alcalin pour 1 mole de bromo-5 hydroxy-4 méthoxy-3 benzaldéhyde. Mais de préférence on utilise un excès d'hydroxyde de métal alcalin ; le plus souvent le rapport molaire hydroxyde de métal alcalin/bromo-5 hydroxy-4 méthoxy-3 benzaldéhyde est compris entre 2 et 5.

La quantité d'eau dans le milieu réactionnel n'est pas critique. Cependant, compte-tenu de la faible solubilité du bromo-5 hydroxy-4 méthoxy-3 benzaldéhyde, le poids de ce composé par rapport au poids d'eau est généralement de 5 à 20 %.

Le catalyseur au cuivre peut être le cuivre métal ou l'un quelconque de ses dérivés organiques ou inorganiques. On peut citer par exemple un oxyde de cuivre, un hydroxyde de cuivre, un sulfate de cuivre, un nitrate de cuivre, un chlorure de cuivre, un acétate de cuivre, un phénate de cuivre ou tout autre composé disponible.

On utilise souvent du cuivre métal car il est facilement mis en oeuvre ; dans le milieu réactionnel ce cuivre métal se transforme au moins partiellement en hydroxyde ou en dérivé du cuivre des différents réactifs présents.

Généralement on utilise un rapport molaire composé du cuivre/bromo-5 hydroxy-4 méthoxy-3 benzaldéhyde de 1 à 10 %. De préférence ce rapport molaire est de 3 à 7 %.

La température à laquelle on opère est généralement située entre 50 et 250°C et de préférence entre 100 et 200°C.

Le bromo-5 hydroxy-4 méthoxy-3 benzaldéhyde utilisé peut être préparé de manière connue en soi. On peut notamment opérer la bromation de la vanilline par le brome en solution acide. On peut par exemple se référer au procédé décrit dans la demande de brevet français publiée sous le numéro 2 177 693.

On peut également bromer la vanilline à l'aide d'un système acide bromhydrique + peroxyde d'hydrogène, comme décrit dans la demande de brevet français publiée sous le numéro 2 557 097 ou à l'aide d'un système brome + peroxyde d'hydrogène comme décrit dans la demande de brevet français publiée sous le numéro 2 557 098.

La deuxième réaction du procédé de l'invention s'effectue sur la solution aqueuse obtenue lors de la première réaction, entre le dihydroxy-4,5 méthoxy-3 benzaldéhyde qu'elle contient et un halogénure d'alkyle inférieur.

Par alkyle inférieur on entend dans le présent texte un groupement alkyle linéaire ou ramifié ayant 1 à 4 atomes de carbone.

Le plus souvent on utilise un halogénure de méthyle ou un halogénure d'éthyle.

Parmi les halogénures, les chlorures, bromures et iodures sont généralement mis en oeuvre et plus spécifiquement encore le chlorure de méthyle, le chloroéthane, le bromure de méthyle et le bromoéthane.

En raison de leur plus faible coût, on préfère utiliser le chlorure de méthyle et le chloroéthane.

Parmi les dialkoxy-4,5 méthoxy-3 benzaldéhydes qui sont obtenus par le procédé de l'invention, le triméthoxy-3,4,5 benzaldéhyde (TMBA) est celui qui a le plus d'utilisations connues, notamment comme intermédiaire pour la préparation de produits pharmaceutiques.

Généralement on met en oeuvre un excès d'halogénure d'alkyle par rapport à la stoechiométrie.

Une variante intéressante du procédé de l'invention consiste à réaliser la réaction d'étherification en milieu biphasique eau/solvant organique non miscible à l'eau.

Le solvant non miscible à l'eau pouvant être utilisé dans la réaction d'éthérification peut être de nature très diverse.

On peut par exemple utiliser un hydrocarbure aromatique tel que le benzène, le toluène, les xylènes ; on peut également utiliser un hydrocarbure aliphatique ou cycloaliphatique tel que l'hexane, l'heptane, l'octane, le décane, le cyclohexane ; les hydrocarbures aromatiques chlorés conviennent également ; ainsi on peut mettre en oeuvre les différentes chlorobenzènes (monochlorobenzène, dichlorobenzènes par exemple), les éthers aliphatiques, aromatiques ou arylaliphatiques tels que par exemple l'oxyde de dibutyle, l'oxyde de dipentyle, l'oxyde de dipropyle, l'oxyde de diisopropyle, l'oxyde d'éthyle et de pentyle, l'anisole, l'oxyde de dibenzyle.

Généralement on utilise un hydrocarbure aromatique ou un éther.

La température de la réaction d'étherification n'est pas critique ; elle a une influence sur la cinétique de la réaction. Généralement on réalise l'éthérification entre 50°C et 150°C. De préférence on opère à un température de 90°C à 120°C.

La pression n'est pas critique. Elle a une incidence sur la cinétique de la réaction. Elle varie habituellement entre la pression atmosphérique et 50 bars.

Le choix d'opérer sous une pression plus ou moins élevée sera conditionnée notamment par la présence ou l'absence de catalyseur. En présence de catalyseur, plus la quantité dudit catalyseur sera élevée, plus la pression pourra être faible, c'est-à-dire par exemple se situer entre la pression atmosphérique et 20 bars.

La pression est habituellement créée par l'halogénure d'alkyle servant à l'éthérification lorsqu'il est gazeux dans les conditions réactionelles.

La présence de catalyseur n'est pas indispensable. Mais généralement il est intéressant de catalyser la réaction, afin d'avoir une cinétique plus élevée et des conditions, notamment de pression, plus douces.

Le catalyseur est choisi parmi les amine et les sels d'ammonium quaternaire.

Toutes les amines primaires, secondaires ou tertiaires peuvent être utilisées.

On peut ainsi utiliser des amines primaires et secondaires aliphatiques, aromatiques, arylaliphatiques, cycloaliphatiques ou hétérocycliques.

A titre d'exemples non limitatifs de telles amines, on peut citer l'éthylamine, la propylamine, la butylamine, l'hexylamine, l'octylamine, la décylamine, la laurylamine, la diméthylamine, la dipropylamine, la diisopropylamine, la dibutylamine, la diisobutylamine, la dihexylamine, l'aniline, la pipéridine, la benzylamine, la méthylaniline, l'éthylaniline, la pyrrolidine, l'imidazole.

Les amines tertiaires utilisables sont également très diverses ; ce sont par exemple des trialkylamines sont les radicaux alkyles peuvent être identiques ou différents et avoir 1 à 24 atomes de carbone ; des cycloalkylalkylamines ; des benzyl-alkylamines ; des phényl-alkylamines; des amines cycliques : ces amines peuvent comporter d'autres fonctions telles que des fonctions éther ou hydroxyle.

On peut citer sans caractère limitatif la triméthylamine, la triéthylamine, la tripropylamine, la tributylamine, l'éthyldiméthylamine, la lauryldiméthylamine, l'octadécyldiméthylamine, la propyldiméthylamine, la pentyldiméthylamine, la benzyldiméthylamine, la trioctylamine, la triéthanolamine, la N,N-diméthylaniline, la butyldiméthylamine, la N,N-diéthylaniline, la N-méthylpipéridine, la N-éthylpipéridine, la N-méthylpyrrolidine, la N-méthylimidazole, la pyridine, la méthyl-3 pyridine, la méthyl-2 pyridine, la méthyl-4 pyridine, la diméthyl-2,4 pyridine, la diméthyl-2,6 pyridine, la triéthylènediamine.

On peut bien entendu utiliser de mélanges industriels (coupes industrielles) disponibles de différentes amines.

D'une manière générale, on préfére utiliser les amines qui sont le plus facilement quaternisables dans les conditions réactionelles.

On peut également utiliser les sels d'ammonium quaternaire comme catalyseur. Ce sont par exemple les halogénures, les hydroxydes, les sulfates d'alkyle, de cycloalkyle ou de benzyle dérivant des amines précédentes.

On préfère généralement les chlorures ou bromures d'ammonium quaternaire.

La quantité de catalyseur utilisé peut varier dans de très larges limites.

Généralement on utilise de 0 à 30 % en moles de catalyseur par rapport au dihydroxy-4,5 méthoxy-3 benzaldéhyde et aux autres hydroxybenzaldéhydes éventuellement présents.

De préférence ce rapport molaire est de 0 à 20 %.

Le catalyseur d'éthérification peut être introduit soit dès la réaction d'hydrolyse, soit seulement lors de la réaction d'éthérification.

Le pH du milieu réactionnel est mainteu à la valeur désirée entre 6 et 12 pendant la durée de la réaction d'éthérification.

Cela se fait par injection ou coulée d'une solution d'une base, qui est généralement l'hydroxyde de métal alcalin utilisé dans la réaction d'hydrolyse du bromo-5 hydroxy-4 méthoxy-3 benzaldéhyde.

De préférence le pH du milieu est maintenu entre 9 et 11.

Le catalyseur au cuivre qui a servi lors de la réaction d'hydrolyse précipite sous forme d'oxyde de cuivre, qui peut être filtré, récupéré et réutilisé dans une nouvelle réaction d'hydrolyse.

Un des avantages du procédé selon l'invention est de permettre une récupération ou un recyclage aisé du catalyseur de la réaction d'hydrolyse, en raison notamment du taux de transformation pratiquement total, lors de la réaction d'éthérification.

Les exemples qui suivent illustrent l'invention.

EXEMPLES 1a et 1b

Exemple 1a :

Dans un réacteur de 1,5 litre en acier inoxydable, muni d'un système de chauffage et d'une agitation, on charge :
- 99,2 g (0,428 mole) de bromo-5 hydroxy-4 méthoxy-3 benzaldéhyde (BHMB)
- 68,8 g de soude en pastilles (1,72 mole)
- 1,6 g de cuivre métal en poudre (0,025 mole)
- 800 cm3 d'eau.

On chauffe sous agitation pendant 2 heures à 150°C afin de réalise l'hydrolyse.

On refroidit ensuite à 100°C ; on ajuste le pH du mélange réactionnel à 10 à l'aide d'acide sulfurique.

On introduit 6,0 g (0,019 mole) de chlorure de tributylbenzylammonium et 150 cm3 de toluène.

On établit une pression de 6 bars de chlorure de méthyle, que l'on maintient constante pendant la durée de l'essai.

Le pH du mélange réactionnel est maintenu à 10 par injection d'une solution aqueuse de soude à 30 % en poids.

Après 2 heures de réaction à 100°C, on refroidit et on soutire le mélange réactionnel final.

On rince le réacteur à l'aide de 100 cm3 d'eau, puis de 100 cm3 de toluène. Ces lavages sont réunis au mélange réactionnel.

On filtre la partie insoluble (oxyde cuivreux) que l'on lave et sèche ; on trouve 1,8 g de $Cu_2O$ (soit la totalité du cuivre engagé).

Les phases aqueuse et organique sont séparées.

On dose par chromatographie liquide haute pression (CLHP) les constituants principaux des deux phases.

```
- Taux de transformation (TT) du BHMB :            100 %
- Rendement (RT) en triméthoxybenzaldéhyde (TMBA)
  par rapport au BHMB transformé :                  80 %.
- RT en diméthoxy-3,4 benzaldéhyde par rapport
  au BHMB transformé :                              14 %.
```

Lors de l'hydrolyse du BHMB il se forme un peu de vanilline comme produit secondaire. Lors de l'éthérification cette vanilline donne du diméthoxy-3,4 benzaldéhyde qui est séparé par distillation et est valorisé.

Exemple 1b :

On charge dans le réacteur décrit précédemment les mêmes quantités des mêmes réactifs que dans l'exemple 1a, mais le cuivre servant de catalyseur est remplacé par le catalyseur récupéré dans l'exemple 1a ($Cu_2O$).

Les conditions opératoires de l'éthérification sont les mêmes que dans l'exemple 1a, à l'exception de la température (110°C au lieu de 100°C) et de la durée (1 h 45 min au lieu de 2 h).

Le traitement du mélange réactionnel final est le même que celui décrit pour 1a et les résultants obtenus sont les suivants :

```
- TT du BHMB :                                  100 %
- RT en TMBA :                                   85 %
- RT en diméthoxy-3,4 benzaldéhyde :             12 %
- Récupération du catalyseur au cuivre : 100 %.
```

EXEMPLE 2

Dans l'appareilliage décrit dans l'exemple 1, on charge :
– 99,2 g (0,428 mole) de bromo-5 hydroxy-4 méthoxy-3 benzaldéhyde (BHMB)
– 66,8 g de soude en pastilles (1,67 mole)
– 1,6 g de cuivre métal en poudre (0,025 mole)
– 6 g de chlorure de tributyl-benzylammonium (0,019 mole)
– 800 cm3 d'eau.
On réalise la réaction d'hydrolyse par chauffage sous agitation pendant 4 heures à 135°C.

On opère l'éthérification comme décrit dans l'exemple 1a pendant 2 heures à 100°C, en maintenant le pH du mélange réactionnel à 10 pendant la durée de l'essai, par injection d'une solution aqueuse de soude à 30 % en poids et en maintenant une pression de 6 bars de chlorure de méthyle.

Le traitement et les dosages du mélange réactionnel final sont les mêmes que ceux décrits dans l'exemple 1a et les résultats obtenus sont les suivants :

```
- TT du BHMB :                                  100 %
- RT en TMBA :                                   85 %
- RT en diméthoxy-3,4 benzaldéhyde               12 %
- Récupération du catalyseur au cuivre : 100 %.
```

EXEMPLE 3

La réaction d'éthérification est effectuée sans catalyseur.
Dans l'appareillage décrit dans l'exemple 1, on charge :
– 99,2 g (0,428 mole) de bromo-5 hydroxy-4 méthoxy-3 benzaldéhyde (BHMB)

5

– 66,8 g de soude en pastilles (1,67 mole)
– 1,6 g de cuivre métal en poudre (0,025 mole)
– 800 cm3 d'eau.
On réalise la réaction d'hydrolyse par chauffage sous agitation pendant 4 heures à 135°C.

Après refroidissement à 100°C et ajustement du pH à 10 comme dans l'exemple 1, on charge 150 cm3 de toluène et on réalise la réaction d'éthérification pendant 4 h 30 min à 100°C, à pH 10 (maintenu par injection de solution aqueuse de soude à 30 % en poids) et en maintenant pendant la durée de l'essai une pression de 10 bars de chlorure de méthyle.

Le traitement et les dosages du mélange réactionnel final sont les mêmes que ceux décrit dans l'exemple 1a et les résultats obtenus sont les suivants :

```
- TT du BHMB :                            100   %
- RT en TMBA :                             80   %
- RT en diméthoxy-3,4 benzaldéhyde :       13,5 %
- Récupération du catalyseur au cuivre : 100   %.
```

EXEMPLE 4

On répète l'exemple 3 avec les mêmes charges, les mêmes conditions opératoires et le même traitement, mais sans ajouter de toluène avant d'effecteur la réaction d'éthérification.
On obtient les résultats suivants :

```
- TT du BHMB :                            100 %
- RT en TMBA :                             80 %
- RT en diméthoxy-3,4 benzaldéhyde :       15 %
- Récupération du catalyseur au cuivre : 100   %.
```

EXEMPLE 5

Dans l'appareillage décrit dans l'exemple 1, on charge 850 cm3 d'une solution aqueuse sodique provenant de l'hydrolyse du bromo-5 hydroxy-4 méthoxy-3 benzaldéhyde et contenant :
– 0,333 mole de dihydroxy-4,5 méthoxy-3 benzaldéhyde (DHMB)
– 0,074 mole de vanilline
– 0,022 mole de cuivre provenant de la catalyse de la réaction d'hydrolyse.
On ajoute 150 cm3 de toluène et 23,9 g (0,074 mole) de bromure de tétrabutylammonium.
On chauffe à 100°C sous agitation et on ajuste le pH à 10.
Cette valeur de pH est maintenue pendant toute la durée de l'essai, par injection d'une solution aqueuse de soude à 30 % en poids.
On établit une pression de 3 bars de chlorure de méthyle et l'on maintient cette valeur pendant tout l'essai.
Après 2 h 30 min de réaction, on refroidit et traite le mélange réactionnel final comme indiqué dans l'exmple 1a.
On obtient les résultats suivants :

```
( TT du DHMB : 100 %

( RT en TMBA :   97 %


( TT de la vanilline :            100   %

( RT en diméthoxy-3,4 benzaldéhyde :  92,5 %


    Récupération du catalyseur au cuivre : 100 %
```

EXEMPLE 6

Dans l'appareillage décrit dans l'exemple 1, on charge 860 cm3 d'une solution aqueuse sodique provenant de l'hydrolyse du bromo-5 hydroxy-4 méthoxy-3 benzaldéhyde et contenant :
– 0,314 mole de dihydroxy-4,5 méthoxy-3 benzaldéhyde (DHMB)
– 0,057 mole de vanilline
– 0,0205 mole de cuivre provenant de la catalyse de la réaction d'hydrolyse.
On ajoute 140 cm3 de toluène et 12,7 g (0,069 mole) de tributylamine.
On chauffe à 105°C sous agitation et on ajuste le pH à 9,5. Cette valeur de pH est maintenue pendant toute la durée de l'essai, par injection d'une solution aqueuse de soude à 30 % en poids.
On établit une pression de 6 bars de chlorure de méthyle et l'on maintient cette valeur pendant toute la durée de l'essai.
Après 2 h 30 min de réaction, on refroidit et traite le mélange réactionnel final comme indiqué dans l'exemple 1a.
On obtient les résultats suivants :

```
( TT du DHMB : 100 %

( RT en TMBA :   99 %


( TT de la vanilline :            100 %

( RT en diméthoxy-3,4 benzaldéhyde : 100 %


    Récupération du catalyseur au cuivre : 100 %
```

EXEMPLE 7

L'exemple 6 est répété avec les mêmes charges et dans les mêmes conditions opératoires, à l'exception de la pression du chlorure de méthyle qui est maintenue à 10 bars au lieu de 6 bars. La durée de la réaction est de 1 h 40 min.
On obtient les résultats suivants :

```
( TT du DHMB : 100 %

( RT en TMBA :   95 %


( TT de la vanilline :            100 %

( RT en diméthoxy-3,4 benzaldéhyde : 100 %


    Récupération du catalyseur au cuivre : 100 %
```

EXEMPLE 8

Dans l'appareillage décrit dans l'exemple 1, on charge 950 g d'une solution aqueuse sodique provenant de l'hydrolyse du bromo-5 hydroxy-4 méthoxy-3 benzaldéhyde et contenant :
– 0,314 mole de dihydroxy-4,5 méthoxy-3 benzaldéhyde (DHMB)
– 0,057 mole de vanilline
– 0,205 mole de cuivre provenant de la catalyse de la réaction d'hydrolyse.
On ajoute 140 cm3 de toluène et 10,6 g (0,034 mole) de chlorure de tributyl-benzylammonium.
On chauffe à 100°C sous agitation et on ajuste le pH à 10.
Cette valeur de pH est maintenue pendant toute la durée de l'essai, par injection d'une solution aqueuse de soude à 30 % en poids.
On établit une pression de 6 bars de chlorure de méthyle et l'on maintient cette valeur pendant tout l'essai.
Après 1 h 15 min de réaction, on refroidit et on traite le mélange réactionnel final comme indiqué dans l'exemple 1a.
On obtient les résultats suivants :

```
( TT du DHMB : 100 %

( RT  en TMBA : 100 %


( TT de la vanilline :              100 %

( RT en diméthoxy-3,4 benzaldéhyde : 100 %


      Récupération du catalyseur au cuivre : 100 %
```

EXEMPLE 9

On répète l'exemple 8 avec les mêmes charges et dans les mêmes conditions opératoires, mais on remplace le toluène par le même volume d'oxyde de diisopropyle.
On obtient les résultats suivants :

```
( TT du DHMB : 100 %

( RT en TMBA : 100 %


( TT de la vanilline :              100 %

( RT en diméthoxy-3,4 benzaldéhyde : 100 %


      Récupération du catalyseur au cuivre : 100 %
```

EXEMPLES 10 à 16

Dans l'appareillage décrit dans l'exemple 1, on charge 640 cm3 d'une solution aqueuse contenant :
– 0,893 mole de dihydroxy-4,5 méthoxy-3 benzaldéhyde (DHMB)
– 0,197 mole de vanilline.
On ajoute 350 cm3 de toluène et la quantité d'amine indiquée dans le tableau ci-après.
On chauffe à 100°C sous agitation et ajuste le pH à 10.
Cette valeur de pH est maintenue pendant toute la durée de l'essai, par injection d'une solution aqueuse de soude à 30 % en poids.
On établit une pression de 4 bars de chlorure de méthyle et l'on maintient cette valeur pendant tout l'essai.
Après 4 h ou 5 h (la durée est indiquée dans le tableau ci-après) de réaction, on refroidit et on traite le mélange réactionnel final comme indique dans l'exmple 1a.

Le tableau ci-après indique les TT de la vanilline et du DHMB ainsi que les RT respectifs en diméthoxy-3,4 benzaldéhyde (DMB) et en triméthoxybenzaldéhyde (TMBA) ainsi qu'en hydroxydiméthoxybenzaldéhydes (HDMB) provenant de l'éthérification d'une seule fonction OH du DHMB).

| EXEMPLES | Amine et quantité | Durée | TT % du DHMB | RT % en TMBA | RT % en HDMB | TT % de la vanilline | RT % en DMB |
|---|---|---|---|---|---|---|---|
| Exemple 10 | triéthanolamine 24 g (0,161 mole) | 5 h | 97 | 80 | 20 | 88 | 96 |
| Exemple 11 | n-octylamine 22 g (0,170 mole) | 4 h | 97 | 83 | 14 | 93 | 95 |
| Exemple 12 | N,N-diméthyl-benzylamine 23 g (0,170 mole) | 5 h | 99 | 85 | 12 | 92 | 97 |
| Exemple 13 | trioctylamine 57 g (0,161 mole) | 4 h | 100 | 99 | 0 | 100 | 100 |
| Exemple 14 | tributylamine 36,5 g (0,197 mole) | 4 h | 100 | 96 | 0 | 100 | 100 |
| Exemple 15 | N,N-diméthyl-aniline 21 g (0,173 mole) | 5 h | 98 | 73 | 21 | 88 | 94 |
| Exemple 16 | triéthylène-diamine 20 g (0,238 mole) | 4 h | 97 | 75 | 20 | 86 | 100 |

## Revendications

1. Procédé de préparation de dialkoxy-4,5 méthoxy-3 benzaldéhyde à partir de bromo-5 hydroxy-4 méthoxy-3 benzaldéhyde, caractérisé en ce que l'on effectue successivement et sans séparation des composés intermédiaires :

— l'hydrolyse du bromo-5 hydroxy-4 méthoxy-3 benzaldéhyde en dihydroxy-4,5 méthoxy-3 benzaldéhyde,

EP 0 277 894 B1

par un hydroxyde de métal alcalin, dans l'eau et en présence d'un catalyseur au cuivre ;
– l'éthérification du dihydroxy-4,5 méthoxy-3 benzaldéhyde obtenu précédemment à l'aide d'un halogénure d'alkyle inférieur, en milieu aqueux et en maintenant le pH entre 6 et 12, éventuellement en présence d'un catalyseur.

2. Procédé selon la revendication 1, caractérisé en ce que le catalyseur au cuivre mis en oeuvre représente de 1 à 10 % en moles de la quantité de bromo-5 hydroxy-4 méthoxy-3 benzaldéhyde.

3. Procédé selon l'une des revendications 1 à 2, caractérisé en ce que dans la réaction d'éthérification on utilise un halogénure de méthyle ou d'éthyle.

4. Procédé selon la revendication 3, caractérisé en ce que l'on utilise un chlorure ou un bromure de méthyle ou d'éthyle.

5. Procédé selon l'une des revendications 1 à 4, caractérisé en ce que l'on utilise un excès d'halogénure d'alkyle inférieur par rapport à la stoechiométrie.

6. Procédé selon l'une des revendications 1 à 5, caractérisé en ce que la réaction d'éthérification est effectuée en milieu biphasique eau/solvant organique non miscible à l'eau.

7. Procédé selon la revendication 6, caractérisé en ce que le solvant organique non miscible à l'eau est choisi parmi les hydrocarbures aromatiques, les hydrocarbures aliphatiques, les hydrocarbures aromatiques chlorés et les éthers aliphatiques, aromatiques ou arylaliphatiques.

8. Procédé selon l'une des revendications 1 à 7, caractérisé en ce que l'on réalise la réaction d'hydrolyse entre 50°C et 250°C et de préférence entre 100°C et 200°C.

9. Procédé selon l'une des revendications 1 à 7, caractérisé en ce que l'on réalise la réaction d'étherification à un pH du mélange réactionnel de 9 à 11.

10. Procédé selon l'une des revendications 1 à 9, caractérisé en ce que l'on opère la réaction d'éthérification entre 50°C et 150°C et de préférence entre 90°C et 120°C.

11. Procédé selon l'une des revendications 1 à 10, caractérisé en ce que la réaction d'éthérification est faite sous une pression comprise entre la pression atmosphérique et 50 bars et de préférence entre la pression atmosphérique et 20 bars.

12. Procédé selon l'une des revendications 1 à 11, caractérisé en ce que l'on opère la réaction d'éthérification en présence d'un catalyseur choisi parmi les amines primaires, secondaires ou tertiaires et les sels d'ammonium quaternaire.

13. Procédé selon la revendication 12, caractérisé en ce que le catalyseur est choisi parmi les amines primaires et secondaires aliphatiques, aromatiques, arylaliphatiques, cycloaliphatiques ou hétérocycliques ; parmi les amines tertiaires telles que des trialkylamines dont les radicaux alkyles peuvent être identiques ou différents et avoir 1 à 24 atomes de carbone ; des cycloalkylalkylamines ; des benzylalkylamines, des phényl-alkylamines ; des amines cycliques ; de telles amines pouvant comporter d'autres fonctions telles que des fonctions éther ou hydroxyle et parmi les sels d'ammonium quaternaires tels que les halogénures, les hydroxydes, les sulfates d'alkyle, de cycloalkyle ou de benzyle dérivant des amines précédentes.

14. Procédé selon l'une des revendications 1, 12 et 13, caractérisé en ce que le rapport molaire entre le catalyseur de la réaction d'éthérification et le dihydroxy-4,5 méthoxy-3 benzaldéhyde et les autres hydroxybenzaldéhydes éventuellement présents, est compris entre 0 et 30 % et de préférence entre 0 et 20 %.

## Claims

1. Process for the preparation of 4,5-dialkoxy-3-methoxybenzaldehyde starting with 5-bromo-4-hydroxy-3-methoxybenzaldehyde, characterised in that the following are performed in sequence and without isolation of the intermediate compounds:
– hydrolysis of 5-bromo-4-hydroxy-3-methoxybenzaldehyde into 4,5-dihydroxy-3-methoxybenzaldehyde by an alkali metal hydroxide, in water and in the presence of a copper catalyst;
– etherification of the 4,5-dihydroxy-3-methoxybenzaldehyde obtained above, using a lower alkyl halide, in an aqueous medium and maintaining the pH between 6 and 12, optionally in the presence of a catalyst.

2. Process according to Claim 1, characterised in that the copper catalyst employed represents from 1 to 10 mol % of the quantity of 5-bromo-4-hydroxy-3-methoxybenzaldehyde.

3. Process according to one of Claims 1 and 2, characterised in that a methyl or ethyl halide is employed in the etherification reaction.

4. Process according to Claim 3, characterised in that a methyl or ethyl chloride or bromide is employed.

5. Process according to one of Claims 1 to 4, characterised in that an excess of lower alkyl halide relative to the stoichiometry is employed.

6. Process according to one of Claims 1 to 5, characterised in that the etherification reaction is carried out

10

in a two-phase medium consisting of water and a water-immiscible organic solvent.

7. Process according to Claim 6, characterised in that the water-immiscible organic solvent is chosen from aromatic hydrocarbons, aliphatic hydrocarbons, chlorinated aromatic hydrocarbons and aliphatic, aromatic or arylaliphatic ethers.

8. Process according to one of Claims 1 to 7, characterised in that the hydrolysis reaction is carried out between 50°C and 250°C and preferably between 100°C and 200°C.

9. Process according to one of Claims 1 to 7, characterised in that the etherification reaction is carried out at a pH of the reaction mixture from 9 to 11.

10. Process according to one of Claims 1 to 9, characterised in that the etherification reaction is carried out between 50°C and 150°C and preferably between 90°C and 120°C.

11. Process according to one of Claims 1 to 10, characterised in that the etherification reaction is carried out at a pressure between atmospheric pressure and 50 bars and preferably between atmospheric pressure and 20 bars.

12. Process according to one of Claims 1 to 11, characterised in that the etherification reaction is carried out in the presence of a catalyst chosen from primary, secondary or tertiary amines and quaternary ammonium salts.

13. Process according to Claim 12, characterised in that the catalyst is chosen from aliphatic, aromatic, arylaliphatic, cycloaliphatic or heterocyclic primary and secondary amines; from tertiary amines such as trialkylamines, the alkyl radicals of which may be identical or different and may contain from 1 to 24 carbon atoms; cycloalkylalkylamines; benzylalkylamines, phenylalkylamines; and cyclic amines; it being possible for such amines to carry other groups such as ether or hydroxyl groups and from amongst quaternary ammonium salts such as the alkyl, cycloalkyl or benzyl halides, hydroxides and sulphates derived from the above amines.

14. Process according to one of Claims 1, 12 and 13, characterised in that the molar ratio of the etherification reaction catalyst to 4,5-dihydroxy-3-methoxybenzaldehyde and the other hydroxybenzaldehydes possibly present is between 0 and 30% and preferably between 0 and 20%.

## Patentansprüche

1. Verfahren zur Herstellung von 4,5-Dialkoxy-3-methoxybenzaldehyd aus 5-Bromo-4-hydroxy-3-methoxybenzaldehyd, dadurch gekennzeichnet, daß man nacheinander und ohne Trennung der Zwischenprodukte
   – die Hydrolyse von 5-Bromo-4-hydroxy-3-methoxybenzaldehyd in 4,5-Dihydroxy-3-methoxybenzaldehyd durch ein alkalisches Metallhydroxid in Wasser und in Gegenwart eines Kupferkatalysators;
   – die Veretherung von 4,5-Dihydroxy-3-methoxybenzaldehyd, das zuvor mit Hilfe eines niederen Alkylhalogenids erhalten wurde, in wäßrigem Milieu und unter Erhaltung des pH-Werts zwischen 6 und 12, gegebenenfalls in Gegenwart eines Katalysators, durchführt.

2. Verfahren nach Anspruch 1, dadurch gekennzeichnet, daß der eingesetzte Kupferkatalysator 1 bis 10 Mol% der Menge des 5-Bromo-4-hydroxy-3-methoxybenzaldehyds ausmacht.

3. Verfahren nach einem der Ansprüche 1 bis 2, dadurch gekennzeichnet, daß man für die Veretherung ein Methyl- öder Ethylhalogenid verwendet.

4. Verfahren nach Anspruch 3, dadurch gekennzeichnet, daß man ein Chlorid oder Bromid von Methyl oder Ethyl verwendet.

5. Verfahren nach einem der Ansprüche 1 bis 4, dadurch gekennzeichnet, daß man das niedere Alkylhalogenid im Überschuß, bezogen auf das stöchiometrische Verhältnis, verwendet.

6. Verfahren nach einem der Ansprüche 1 bis 5, dadurch gekennzeichnet, daß man die Veretherung im zweiphasigen Medium Wasser/organisches, mit Wasser nicht mischbares Lösungsmittel durchführt.

7. Verfahren nach Anspruch 6, dadurch gekennzeichnet, daß das mit Wasser nicht mischbare organische Lösungsmittel ausgewählt ist aus aromatischen Kohlenwasserstoffen, aliphatischen Kohlenwasserstoffen, chlorierten aromatischen Kohlenwasserstoffen und aliphatischen, aromatischen oder arylaliphatischen Ethern.

8. Verfahren nach einem der Ansprüche 1 bis 7, dadurch gekennzeichnet, daß man die Hydrolysereaktion zwischen 50°C und 250°C und vorzugsweise zwischen 100°C und 200°C durchführt.

9. Verfahren nach einem der Ansprüche 1 bis 7, dadurch gekennzeichnet, daß man die Veretherung bei einem pH des Reaktionsgemisches von 9 bis 11 durchführt.

10. Verfahren nach einem der Ansprüche 1 bis 9, dadurch gekennzeichnet, daß man die Veretherung zwischen 50°C und 150°C und vorzugsweise zwischen 90°C und 120°C durchführt.

11. Verfahren nach einem der Ansprüche 1 bis 10, dadurch gekennzeichnet, daß die Veretherung zwischen Atmosphärendruck und 50 bar und vorzugsweise zwischen Atmosphärendruck und 20 bar durchgeführt wird.

12. Verfahren nach einem der Ansprüche 1 bis 11, dadurch gekennzeichnet, daß man die Veretherungsreaktion in Gegenwart eines Katalysators, ausgewählt aus primären, sekundären oder tertiären Aminen und aus quaternären Ammoniumsalzen, ausführt.

13. Verfahren nach Anspruch 12, dadurch gekennzeichnet, daß der Katalysator ausgewählt ist aus aliphatischen, aromatischen, arylaliphatischen, cycloaliphatischen oder heterocyclischen primären und sekundären Aminen; aus tertiären Aminen wie Trialkylaminen, deren Alkylreste identisch oder verschieden sein und 1 bis 24 Kohlenstoffatome haben können;Cycloalkylalkylaminen;Benzylalkylaminen, Phenylalkylaminen; cyclischen Aminen; wobei solche Amine andere funktionnelle Gruppen wie Ether- oder Hydroxylgruppen haben Können, und aus quaternären Ammoniumsalzen wie den Alkyl-, Cycloalkyl- oder Benzylhalogeniden, -hydroxiden oder -sulfaten, die von den zuvor gennanten Aminen abgeleitet sind.

14. Verfahren nach einem der Ansprüche 1, 12 und 13, dadurch gekennzeichnet, daß das Molverhältnis zwischen dem Katalysator für die Veretherung und dem 4,5-Dihydroxy-3-methoxybenzaldehyd und anderen gegebenenfalls vorhandenen Hydroxybenzaldehyden zwischen 0 und 30 % und vorzugsweise zwischen 0 und 20 % liegt.